# EUROPÄISCHE PATENTSCHRIFT

(11) **EP 2 526 897 B1**
(45) Veröffentlichungstag und Bekanntmachung des Hinweises auf die Patenterteilung: **16.03.2016**
(21) Anmeldenummer: 12005881.3
(22) Anmeldetag: 14.09.2006
(51) Int. Cl.: A61F 2/24

(54) **Biologische oder künstliche Klappenprothese zur Verwendung im menschlichen und/oder tierischen Körper zum Ersatz einer Organklappe oder Gefässklappe**
Biological or artificial valve prosthetic for use in human and/or animal bodies for replacing an organ valve or vessel valve
Prothèse de valve artificielle ou biologique destinée à une utilisation dans un corps humain et/ou animal en remplacement de la valve d'un organe ou d'un vaisseau

(30) Priorität: 14.09.2005 DE 102005044009
(43) Veröffentlichungstag der Anmeldung: 28.11.2012
(62) Teilanmeldung aus: 06805720.7
(73) Patentinhaber: Vueklar Cardiovascular Ltd., Glentirranmuir Kippen Stirling FK8 2HU (GB)
(72) Erfinder: Melzer, Andreas, 65227 Niedernhausen (DE); Immel, Erwin, 45897 Gelsenkirchen (DE)
(74) Vertreter: Von Rohr Patentanwälte Partnerschaft mbB

(56) Entgegenhaltungen:
- EP-A2- 1 092 985
- US-A1- 2005 049 692
- US-B1- 6 767 360

## Beschreibung

Die Erfindung betrifft eine biologische oder künstliche Klappenprothese zur Verwendung im menschlichen oder tierischen Körper zum Ersatz einer Organklappe oder Gefäßklappe, insbesondere Herz- oder Venenklappenprothese, mit einem Stent oder stentlos, mit einem tragenden Klappengerüst, mit wenigstens einer Klappe und mit mindestens einer Leiterschleife, die die Induktivität eines elektrischen Resonanzschwingkreises ausbildet.

Venenklappen befinden sich biologisch in den tiefen Beinvenen. Sie dienen der Verhinderung des Rückflusses des venösen Blutes in die Gliedmaßen. Durch Kompression der Venen beim Anspannen der Muskeln wird das Blut in Richtung des Herzens transportiert. Die Klappen können undicht (insuffizient) werden oder ihre Funktion durch Anlagerung von geronnenem Blut (Beinvenenthrombose) verlieren. Venenklappenprothesen sind derzeit in der Entwicklung und bestehen in der Regel aus einem Stentgerüst aus selbstexandierendem Material, wie beispielsweise Nitinol oder einem Stahlgerüst, in welches entweder eine Klappe aus Polymer oder ein Dünnfilm aus Nitinol oder ein Metallgeflecht eingearbeitet sind. Die Klappenfunktion kann ggf. durch die Anlage einer Gewebemanschette mit Einengung der Vene chirurgisch wiederhergestellt werden.

Die Herzklappen befinden sich innerhalb des Herzens und trennen die Vorhöfe von den Herzkammern, wobei sie als Rückschlagventil dafür sorgen, dass das Blut in die richtige Richtung fließt. Das Herz hat vier Klappen, wovon jeweils zwei dem rechten und dem linken Herzen angehören. Das Blut gelangt aus den venösen Gefäßen in den Vorhof oder die Vorkammer, von dort über eine Klappe in die Herzkammer und von dort wiederum über eine Klappe in die Gefäße des großen oder kleinen Kreislaufs. Die Herzklappen bestehen aus dünnen, stabilen Bindegewebsschichten, die taschenförmig (Aortenklappe und Pulmonalklappe) bzw. segelförmig (Mitralklappe und Trikuspidalklappe) geformt sind. Sie sind wie der übrige Herzinnenraum mit der glatten Innenhaut des Herzens (Endokard) überzogen.

Bei erheblicher Einschränkung der Klappenfunktion durch angeborene oder erworbene Defekte, wie Klappeninsuffizienz oder Klappenstenose, ist der Ersatz der Herzklappe durch eine Prothese erforderlich. Es gibt zwei Arten von Herzklappenprothesen, nämlich die biologische und die mechanische Herzklappe. Die biologischen Herzklappen werden auch stentlose Klappenprothesen genannt, da sie nicht über ein stabilisierendes Gerüst verfügen. Die Klappen sind präparierte Schweineherzklappen oder werden aus dem Gewebe des Rinderherzbeutels oder der Submusosa des Schweinedünndarms hergestellt oder aus menschlichen Endothelzellen auf Kollagenfasern oder Kunststoff-, Metallnetzen/-folien entsprechend der Form und Funktion der menschlichen Herzklappe gezüchtet. Diese Prothesen machen in den meisten Fällen keine lebenslange orale Antikoagulation zur Blutverdünnung notwendig, sondern nur eine temporäre Gerinnungshemmung in den ersten Monaten nach der Operation.

Mechanische Klappen bestehen aus Metall und/oder Kunststoff. Sie erfordern eine lebenslange Antikoagulation, sind aber in den meisten Fällen nahezu unbegrenzt haltbar. Die Klappen sind von einem Gewebegeflecht umhüllt, mit dem diese in das Lager der ehemaligen defekten Herzklappe eingenäht werden.

Die Operationen zum Ersatz defekter Herzklappen mit Prothesen werden in der Herzchirurgie unter Herbeiführung eines (kardioplegischen, diastolischen) Herzstillstandes durchgeführt, um einen blutfreien, ruhigen Operationsitus zu erhalten. In diesem Zustand werden verschiedenste Arten von Herzklappen eingenäht. Als Ausgleich zu diesem Herzstillstand muß zum einen eine Herzlungenmaschine den Kreislauf aufrechterhalten, zum anderen muß die Hauptschlagader (Aorta) abgeklemmt werden. Diese Maßnahmen sind mit erheblichen Nebenwirkungen und Komplikationen verbunden.

Die aktuellen Entwicklungen gehen daher in die Richtung einer Implantation von faltbaren Herzklappenprothesen, die über Katheter eingeführt und plaziert werden. Diese Prothesen basieren auf einem stentartigen Gerüst, in das entweder eine biologische Klappe oder eine Polymer-Klappe eingebaut ist. Die Implantation erfolgt durch Ballondilatation oder selbstexpandierend. Von Nachteil ist jedoch, dass die Stentgerüste der faltbaren, bekannten Herzklappen-Prothesen eine MRT-Visualisierung und damit eine sehr einfache Überprüfung der Klappenfunktion beeinträchtigen.

Eine perkutan implantierbare Herzklappenprothese kann das Risiko, das bei einer offenen Operation vorhanden ist, deutlich minimieren, jedoch ist die präzise Positionierung unter Röntgenkontrolle nicht möglich, da das Weichgewebe des Herzens nicht dargestellt wird und nur mittels Kontrastmittelgabe der blutdurchflossene Raum erscheint. Eine Implantation durch MRT-Bildgebung wäre vorteilhaft, da sowohl das strömende Blut als auch das Herzgewebe ohne Kontrastmittel dargestellt werden.

Die derzeit am Markt verfügbaren faltbaren Herzklappen haben nach dem heutigen Stand der Technik noch keine optimalen Eigenschaften, denn die Funktion der Implantate kann nur eingeschränkt, invasiv durch den Einsatz von Kathetern, Röntgenstrahlung und Kontrastmitteln mit erheblichen Risiken und Belastungen für die Patienten und das medizinische Personal kontrolliert werden. Sowohl beim Setzen des Implantats als auch bei den erforderlichen Nachuntersuchungen sind häufig lange Durchleuchtungszeiten erforderlich. Diese betragen zwischen 10 und 15 Minuten, können aber auch durchaus 60 Minuten und mehr umfassen. Aus den langen Expositionszeiten resultieren Nebenwirkungen wie die Entwicklung von Strahlengeschwüren (Ulceration), die zu Hautkrebs führen können. Die langen Durchleuchtungszeiten und entsprechend hohe Dosiswerte haben auch dazu geführt, dass Patienten, die sich einer kardiologischen Intervention unterzogen haben, Strahlenschäden erlitten haben. Das Bundesamt für Strahlenschutz hat diesbezüglich in einer Untersuchung festgestellt, dass die Reduktion der Strahlenexposition in der medizinischen Diagnostik von besonderer Bedeutung ist.

Untersuchungstechniken der Magnet-Resonanz-Tomographie (MRT) können zur Verminderung röntgendiagnostischer und nuklearmedizinischer Untersuchungsverfahren erheblich beitragen. Neben der fehlenden Strahlenbelastung hat die Magnet-Resonanz-Tomographie im Vergleich zu röntgentechnischen Verfahren wie der Computertomographie und der Durchleuchtung weitere Vorteile. Sie bietet einen hervorragenden Weichteilkontrast und als einziges aller Schichtbildverfahren die völlig freie Schichtpositionierung. Ortsaufgelöste spektrometrische Techniken erlauben eine funktionelle Analyse biochemischer Vorgänge im menschlichen Körper. Ebenso können Gefäße ohne Verwendung von Kontrastmitteln direkt im Operationsgebiet dargestellt werden. Im offenen Magnet-Resonanz-Tomographen hat der Arzt Zugang zum Patienten und kann so unter direkter MRT-Kontrolle Punktionen und Operationen vornehmen. Katheterisierungen sind auch in geschlossenen Hochfeldgeräten mit moderner Bildgebung gut durchzuführen.

Eine Herzklappenprothese der eingangs genannten Art ist bereits aus der EP 1 092 985 A2 bekannt, aus der auch das MR-Bildgebungsverfahren hervorgeht, worauf ausdrücklich Bezug genommen wird. Die bekannte Herzklappenprothese weist ein äußeres Klappengerüst auf, an dem die eigentliche Herzklappe beweglich gelagert ist. Zur Ausbildung des Resonanzschwingkreises sind sowohl Spulen als auch Kondensatoren am ringförmigen Traggerüst vorgesehen. Vorteilhaft bei der bekannten Herzklappenprothese ist, dass sich diese hervorragend durch das MR-Bildgebungsverfahren darstellen läßt. Von Nachteil ist, dass diese Prothese nicht perkutan implantierbar ist.

Konventionelle faltbare Herzklappen lassen sich zwar perkutan implantieren, die Darstellung im Magnet-Resonanz-Tomographen hat gegenwärtig jedoch folgende entscheidende Nachteile:
- eine starke Störungs- und Artefaktempfindlichkeit aufgrund der verwendeten Metalle,
- das Innere eines Metall-Implantats ist aufgrund des Faraday-Käfigeffektes abgeschirmt und nur schwer sichtbar,
- metall- oder kunststoffbasierte Klappenmechanismen sind entweder nicht signalgebend oder führen zu Magnetfeldinhomogenisierung,
- in bewegten Metallkomponenten des Klappenmechanismus im starken Magnetfeld können unerwünschte induktive Effekte auftreten,
- die biologischen Klappen bestehen überwiegend aus nicht- bis schwachsignalgebenden kollagenen Fasern und wenigen Zellen, so dass eine direkte MRT-Bildgebung erschwert ist,
- die zeitliche und örtliche Auflösung zur vollständigen Diagnostik der Klappe und ihrer Funktionen sind unzureichend.

Aufgrund der vorstehenden Probleme sind konventionelle faltbare Herzklappen, die üblicherweise aus Edelstahllegierungen oder Nitinol bestehen, für MRT-Untersuchungen der Klappenfunktionen und der korrekten Lagen der Prothese nicht oder nur unzureichend einsetzbar.

Aufgabe der vorliegenden Erfindung gemäß Anspruch 1 ist es daher, eine Klappenprothese der eingangs genannten Art zur Verfügung zu stellen, bei der die zuvor beschriebenen Nachteile vermieden werden.

Die vorgenannte Aufgabe ist bei einer Klappenprothese der eingangs genannten Art erfindungsgemäß dadurch gelöst, dass die wenigstens eine Leiterschleife das Klappengerüst und/oder die Klappe und/oder tragende Bereiche des Klappengerüstes und/oder tragende Bereiche der Klappe der Prothese ausbildet. Die erfindungsgemäße Ausgestaltung bietet eine Reihe von erheblichen Vorteilen, wobei die wenigstens eine Leiterschleife eine Mehrzahl von Funktionen übernimmt. Zunächst einmal bildet die Leiterschleife den MR-Schwingkreis und stellt damit die MR-Funktion sicher. Darüber hinaus bildet die Leiterschleife das Klappengerüst oder zumindest tragende Bereiche des Klappengerüstes zur Halterung der Klappe. Außerdem kann die Leiterschleife auch die Klappe oder tragende Bereiche der Klappe ausbilden. Da die Leiterschleife bei der erfindungsgemäßen Prothese eine tragende Funktion übernimmt, ist es über die Leiterschleife auch möglich, die Durchgangsöffnung der Prothese offenzuhalten. Schließlich ergibt sich insbesondere dann, wenn die Leiterschleife zusätzlich Verankerungsmittel aufweist, eine Verankerungs- bzw. Befestigungsfunktion.

Bevorzugt ist bei der Erfindung vorgesehen, dass der Stent und/oder das Klappengerüst außenseitig, das heißt an der äußeren umlaufenden Mantelfläche, Zellen unterschiedlicher Größe aufweist. Damit ergibt sich außenseitig eine Gitter- oder Netzform, wobei dann eine Vielzahl von gleichgroßen und auch unterschiedlich großen Zellen bzw. Öffnungen oder Fenstern vorgesehen sein können.

Darüber hinaus ist es dadurch, dass die Leiterschleife das Klappengerüst bildet, sehr einfach möglich, die Prothese insgesamt falt- bzw. expandierbar und damit perkutan implantierbar auszubilden.

Letztlich ergibt sich durch die Erfindung damit eine Klappenprothese aus mindestens einem geschlossenen Schwingkreis mit einer Induktivität und vorzugsweise einer Kapazität, die in zusammengefalteter Anordnung perkutan implantierbar ist, wobei eine optimale Stabilisierung und Verankerung der Prothese mittels der Schwingkreiskonstruktion und -geometrie erreicht wird. Der elektrische Schwingkreis erzeugt dabei eine Signalantwort, die mittels mindestens einer externen Empfangsspule detektiert und ortsaufgelöst dargestellt wird, so dass die MR-Bildgebung der Klappe und ihrer Funktion verbessert wird. Darüber hinaus ist die erfindungsgemäße Klappenprothese in besonderem Maße dazu geeignet, unter MR-Bildgebung zu implantieren, da die Entfaltung wie auch die Positionierung und Verankerung präzise bestimmt werden können.

Zur Verankerung der Leiterschleife bzw. des Schwingkreises bietet es sich an, Verankerungsmittel beispielsweise in Form von Ösen, Haken od. dgl. vorzusehen. Günstig ist es in diesem Zusammenhang, die Verankerungsmittel einstückig mit der Leiterschleife, auszubilden, so dass zur Verankerung keine weiteren Bauteile erforderlich sind. Grundsätzlich ist es allerdings auch möglich, die Ösen, Haken od. dgl. als separate Bauteile auszubilden, die dann an dem Spulendraht nicht verschiebbar befestigt sind. Im Zusammenhang mit den Verankerungsmitteln ist von Bedeutung, dass diese im komprimierten Zustand der Klappenprothese nicht über diese überstehen und erst nach der Entfaltung, wenn sich die Klappenprothese an der Implantationsstelle befindet, wirksam werden und zum Befestigen der Prothese am umgebenden Gewebe genutzt werden können. Durch die Integration und unmittelbare Nähe der Verankerungen zum Schwingkreis ist insbesondere die korrekte Positionierung der Prothese unter MRT sowie die nachfolgende Diagnostik in bezug auf Undichtigkeit des Klappenlagers verbessert.

In besonderem Maße eignet sich die vorliegende Erfindung im Zusammenhang mit biologischen, stentlosen Klappenprothesen, insbesondere heterologe von einem Tier, wie von der Herzklappe oder dem Herzbeutel eines Schweins oder dem Herzbeutel eines Rinds, oder homologe und isologe von menschlichen Geweben und Zellen, wobei die Leiterschleife in diesen Fällen von der biologischen Herzklappe umschlossen ist. Letztlich beinhaltet in diesem Falle die biologische Herzklappe die Leiterschleife und damit den Schwingkreis. Dabei versteht es sich in diesem Zusammenhang, dass die Leiterschleife eine der natürlichen Herzklappe angepaßte Form aufweist, wobei die Klappenansätze oder -flügel vom Spulendraht in mindestens einer Windung umfaßt werden und die Basis der Herzklappe von einer Zylinderspule umschlossen ist.

Denn gerade in Verbindung mit einer biologischen Herzklappe bietet es sich an, dass bioresorbierbare Anteile, die die Klappenprothese für die Implantation stabilisieren und abdichten, vorgesehen sind. Dabei ist dann die wenigstens eine Leiterschleife zur Implantation in einem biologischen Anteil der Klappenprothese vorgesehen und verbleibt dort, nachdem die bioresorbierbaren Anteile im Körper abgebaut sind. Die bioresorbierbaren Anteile dienen damit lediglich der Stabilisation der Klappenprothese bis zum Zeitpunkt unmittelbar nach der Implantation. Die eingewachsene Herzklappe verfügt jedoch weiterhin über die vorteilhaften Eigenschaften für die MRT-Bildgebung, um langfristig auftretende Defekte wie Verschleiß, Gewebereaktion, optimal im MRT darzustellen und rechtzeitig eine Therapie oder einen Ersatz vornehmen zu können.

Während es grundsätzlich möglich ist, die Prothese ohne Stent auszubilden, versteht es sich, dass auch Prothesen mit Stent möglich sind. Hierbei ist dann erfindungsgemäß vorgesehen, dass das Klappengerüst und die Klappe in einem selbst entfaltbaren oder mechanisch, insbesondere hydraulisch bzw. fluidisch expandierbaren Stent durch die Leiterschleife zumindest teilweise derart fixiert sind, dass bei der Komprimierung und anschließenden Entfaltung sowohl das Klappengerüst und die Klappe als auch die Leiterschleife in die gewünschte Form, Position und Geometrie zurückkehren, wobei gleichzeitig die notwendige Abdichtung der Prothese im Lager erreicht werden kann.

Gemäß der Erfindung ist es in diesem Zusammenhang, dass der gesamte Klappenmechnismus über die Leiterschleife nicht nur gehalten ist, sondern dass die Leiterschleife gleichzeitig zur Befestigung der Klappe am Stent dient. Hierbei sind dann zumindest Bereiche der Leiterschleife aus dem Stent herausgeführt. Diese Bereiche können dabei auch der Verankerung im umgebenden Gewebe dienen und somit Verankerungsmittel darstellen.

Bevorzugt kann vorgesehen sein, dass das Gerüst mehrere Resonanzschwingkreise mit unterschiedlichen Resonanzfrequenzen mit jeweils einer Leiterschleife ausbildet oder dass mehrere Resonanzschwingkreise mit unterschiedlichen Resonanzfrequenzen mit nur einer Leiterschleife miteinander gekoppelt sind. Hierdurch wird nicht nur die Tragfunktion des Klappengerüstes verbessert, der Herzklappenersatz kann damit auch in unterschiedlichen MRTs eingesetzt werden. Bevorzugt ist es in diesem Zusammenhang, dass das Klappengerüst mehrere unterschiedliche Spulenformen, d. h. unterschiedliche Spulengeometrien wie beispielsweise Zylinder, Sattel- und Birdcagespulen aufweist.

Im Übrigen kann mindestens eine Spule des Resonanzschwingkreises auch eine Mäanderstruktur aufweisen. Von Vorteil ist es im Übrigen auch, wenn die Leiterschleife bzw. die Leiterbahn selbst mäanderförmig ausgebildet ist. Durch diese Ausbildung ergibt sich eine gezielte und definierte Faltbarkeit und Expansion, wobei die Fläche der Spule erhöht werden kann und die Elastizität im Lagergewebe verbessert wird. Gleichzeitig kann durch diese Ausgestaltung Ermüdungsbrüchen entgegengewirkt werden.

Da die Form der Leiterschleife grundsätzlich beliebig gewählt werden kann, bietet es sich an, dass bei einer biologischen Herzklappe die Leiterschleife die Form einer natürlichen Herzklappe hat und wenigstens zwei Schlaufen der Leiterschleife vorgesehen sind, die mit ihren Flächen eine Zylinderform bilden, die natürliche Klappenform umschließen und in den Bereichen zwischen den Schlaufen die Abgänge der Herzkranzgefäße freihalten.

Um eine kompakte Prothese zu erhalten, bietet es sich an, dass die zum Resonanzschwingkreis gehörende weitere Kapazität auf und/oder in der Klappenprothese befestigt bzw. angebracht ist und/oder in das Design der Klappenprothese integriert ist.

Dabei ist es im Zusammenhang mit der Verankerung so, dass die Induktivität und/oder die Kapazität des Resonanzschwingkreises zumindest teilweise zur Integration in das die Klappenprothese umhüllende Gewebe zur Annaht, Verankerung und/oder Abdichtung vorgesehen ist.

Zur Einstellung einer externen parasitären Kapazität ist die Leiterschleife mit einem Nichtleiter als Isolationsschicht, insbesondere mit Kunststoff und/oder Keramik, beschichtet. Damit läßt sich dann zum einen über die Auswahl und Technik der Beschichtung und zum anderen über die Dicke der Beschichtung, die mindestens 1 Nanometer aufweist, vorzugsweise aber zwischen 1 Nanometer bis 200 Mikrometer liegt, die gewünschte parasitäre Kapazität einstellen. Darüber hinaus wird durch die Isolierungsschicht und den Abstand zwischen den Schlaufen der wenigstens einen Leiterbahn eine interne Kapazität ausgebildet, wobei sich deren Größe wiederum durch die Art der Isolierungsschicht und den Abstand der einzelnen Schlaufen einstellen läßt.

Die äußere Beschichtung bzw. Isolationsschicht besteht dabei vorzugsweise aus Teflon, Polyester, Polyurethan, Parylene oder anderen geeigneten Polymeren. Neben der Isolierfunktion und der Ausbildung der parasitären und internen Kapazität kann die Isolationsschicht die zusätzliche Aufgabe haben, eine weitere zum Resonanzschwingkreis gehörende Spule und/oder wenigstens eine Kapazität auf und/oder in dem Gerüst der Prothese zu befestigen bzw. anzubringen.

Im Übrigen kann es sich auch anbieten, die Leiterschleife bzw. Spule auf dem Klappengerüst bzw. der Klappenprothese mittels einer Schicht zu befestigen bzw. anzubringen. So kann die Klappenprothese bzw. das Klappengerüst mit einem entsprechenden Material beschichtet werden.

Letztlich ist der Resonanzschwingkreis der erfindungsgemäßen Herzklappenprothese derart ausgebildet, dass der Schwingkreis eine Resonanzfrequenz, insbesondere im hochfrequenten Bereich, nämlich im Bereich zwischen 8 bis 400 MHz, besitzt, die der Frequenz eines äußeren Magnetfeldes, insbesondere eines MR-Tomographen entspricht.

Grundsätzlich kann vorgesehen sein, dass die Leiterschleife aus einem Draht oder einem Rohr oder aus Blech geschnitten aus einem Material mit einer geringen magnetischen Suszeptibilität, wie beispielsweise Nitinol oder einer guten elektrischen Leitfähigkeit, wie ein Edelmetall oder eine Edelmetallegierung oder aus einem Material mit einer geringen magnetischen Suszeptibilität und einer guten elektrischen Leitfähigkeit, wie beispielsweise Tantal, hergestellt ist. Alternativ ist es aber auch möglich, dass die Leiterschleife einen nicht leitenden Leiterträger aufweist, der mittels eines leitfähigen Materials, insbesondere Gold, Platin, Tantal und/oder leitfähigen Legierungen, beschichtet ist. Im Übrigen ist es aber auch möglich, dass der Leiterträger elektrisch leitfähig ist und zur Verbesserung seiner elektrischen Leitfähigkeit mit einem entsprechenden Material beschichtet ist. Günstig ist es, die Leiterschleife durch eine Beschichtung der Klappenprothese mit einem leitenden Material zu realisieren. Bevorzugt erfolgt das Aufbringen der Beschichtung über eine Bedampfung oder ein Besputtern (PVD oder CVD).

Eine geringe magnetische Suszeptibilität liegt bei Materialien vor, die eine Dielektrizitätskonstante von sehr viel größer als 1 haben. So liegt beispielsweise die Dielektrizittskonstante der magnetischen Suszeptibilität von Nitinol bei 3· 10⁶. Demgegenüber weisen Materialien mit einer guten elektrischen Leitfähigkeit in [S/m] Werte auf, die sehr viel größer als 1 sind. So beträgt die elektrische Leitfähigkeit von Titan 2,34 · 10⁶ S/m, während die von Kupfer zwischen 35 bis 58 · 10⁶ S/m liegt.

Im Zusammenhang mit dem Aufbringen der Beschichtung bietet es sich insbesondere bei Polymeren, Keramik, Nickeltitan und Titan als Material des Leiterträgers an, eine Basisschicht auf dieses Material aufzubringen, um die Haftung der Beschichtung zu verbessern. Bei der Basisschicht handelt es sich dann letztlich um einen Haftvermittler.

Zusätzlich zu der zuvor beschriebenen parasitären und/oder inneren Kapazität bietet es sich im Übrigen an, dass eine zusätzliche Kapazität mittels eines Kondensators realisiert wird. Hierdurch ist es dann nicht erforderlich, besondere Formen oder besondere Beschichtungen der Leiterschleife zu realisieren, um vorgegebene innere und parasitäre Kapazitäten zu erzielen.

Zur Erleichterung der Implantation und um sowohl das Auffalten als auch die Funktion der erfindungsgemäßen Klappenprothese verfolgen zu können, ist bei einer faltbaren bzw. entfaltbaren Klappenprothese vorgesehen, dass diese derart ausgebildet ist, dass beim Entfalten die Resonanzfrequenz durch die Änderung der Spulengeometrie des Schwingkreises verändert wird und auf die Resonanzfrequenz des MR-Tomographen einstellbar ist.

Darüber hinaus betrifft die vorliegende Erfindung eine perkutan implantierbare und expandierbare, biologische oder künstliche Klappenprothese zur Verwendung im menschlichen und/oder tierischen Körper zum Ersatz einer Organklappe oder Gefäßklappe, mit mindestens einem mit einer Klappe versehenen Klappenmechanismus, und mit einem Stent mit einer Hülse aus biokompatiblem Material, wobei der Stent in der Funktionshöhe der Klappe von wenigstens einer Leiterschleife, die die Induktivität eines elektrischen Resonanzschwingkreises ausbildet, umschlossen ist.

Im Gegensatz zu der zuvor beschriebenen Ausführungsform wird nicht die Klappenprothese selbst bzw. das Klappengerüst oder aber die Klappe von der Leiterschleife gebildet. Bei dieser Ausführungsform ist es so, dass der Resonanzschwingkreis in Form der wenigstens einen Leiterschleife um den Stent herum in Funktionshöhe der Klappe angebracht ist.

Bei der vorgenannten Ausführungsform bestehen verschiedene grundsätzliche Alternativen der Anordnung der Leiterschleife. Zum einen kann die Leiterschleife um das Traggerüst des Stents angeordnet sein. Da das Traggerüst des Stents die MR-Funktion jedoch beeinträchtigen kann, bietet es sich zum anderen besonders an, dass das Traggerüst des Stents oder tragende Bereiche des Stentgerüstes von der Leiterschleife gebildet werden. Eine negative Beeinflussung der Resonanzfrequenz durch ein separates Traggerüst kann in diesem Fall nicht auftreten. Außerdem ist es möglich, dass die Leiterschleife zur Befestigung des Klappenmechanismus im Stent dient und dazu zumindest teilweise aus dem Stent herausgeführt ist. Schließlich kann über die Leiterschleifer bzw. entsprechende Verankerungsmittel der Leiterschleife eine Verankerung des Stents im Klappenlager erzielt werden. Erkennbar hat auch hierbei die Leiterschleife dann eine Mehrfachfunktion.

Im Zusammenhang mit der zuvor beschriebenen Ausführungsform mit Stent versteht es sich, dass die diesbezügliche Klappenprothese alle vorgenannten Merkmale für sich oder aber in Kombination aufweisen kann, die zuvor bei der eingangs genannten Klappenprothese beschrieben worden sind.

SchHeßlich wird ein MRT-Verfahren beschrieben, wobei in einem lokal begrenzten Bereich innerhalb und/oder außerhalb der Klappenprothese mittels eines geschlossenen Schwingkreises mit einer Induktivität, dessen Resonanzfrequenz im Wesentlichen gleich der Resonanzfrequenz der eingestrahlten hochfrequenten Strahlung ist, wobei eine veränderte Signalantwort erzeugt wird, und wobei der Bereich mit veränderter Signalantwort von mindestens einer Empfangsspule als Signalantwort detektiert ausgewertet zeit- und ortsaufgelöst unter Verwendung der Atemtriggerung und/oder der EKG-Triggerung dargestellt wird.

Das vorgenannte Verfahren ermöglicht die zeitliche Auflösung und Steuerung der Aquisition nach EKG- und Atemtriggerung des zu behandelnden Patienten.

Nachfolgend werden Ausführungsbeispiele der Erfindung anhand der Zeichnung erläutert. Dabei zeigt
- Fig. 1: eine perspektivische, schematische Ansicht einer biologischen Klappenprothese,
- Fig. 2: perspektivische, schematische Ansicht von weiteren Ausführungsformen biologischer Klappenprothesen,
- Fig. 3: eine perspektivische, schematische Ansicht einer künstlichen Klappenprothese mit einer als Deckel ausgebildeten Klappe auf Basis einer Solenoidspule,
- Fig. 4: eine der Fig. 3 entsprechende Ansicht einer Klappenprothese mit einer als Kugel ausgebildeten Klappe,
- Fig. 5: eine der Fig. 3 entsprechende Ansicht einer Klappenprothese mit einer als Doppelklappe ausgebildeten Klappe,
- Fig. 6: eine perspektivische, schematische. Ansicht einer künstlichen Klappenprothese mit einer als Deckel ausgebildeten Klappe auf Basis einer Sattelspule,
- Fig. 7: eine der Fig. 6 entsprechende Ansicht einer künstlichen Klappenprothese mit einer als Kugel ausgebildeten Klappe,
- Fig. 8: eine der Fig. 6 entsprechende Ansicht einer künstlichen Klappenprothese mit einer als Doppelklappe ausgebildeten Klappe,
- Fig. 9: eine perspektivische, schematische Ansicht einer künstlichen Klappenprothese mit einer als Deckel ausgebildeten Klappe auf Basis einer Solenoidspule mit einer mäanderförmig geformten Leiterschleife,
- Fig. 10: eine der Fig. 9 entsprechende Ansicht einer künstlichen Klappenprothese mit einer als Kugel ausgebildeten Klappe,
- Fig. 11: eine der Fig. 9 entsprechende Ansicht einer künstlichen Klappenprothese mit einer als Doppelklappe ausgebildeten Klappe,
- Fig. 12: eine perspektivische, schematische Ansicht einer biologischen Klappenprothese in einem Stent mit einer Sattelspule,
- Fig. 13: eine perspektivische, schematische Ansicht einer künstlichen Klappenprothese in einem Stent mit einer Sattelspule,
- Fig. 14: eine perspektivische, schematische Ansicht einer künstlichen Klappenprothese in einem Stent mit einer als Kugel ausgebildeten Klappe,
- Fig. 15: eine der Fig. 13 entsprechende Ansicht einer künstlichen Klappenprothese mit einer als Doppelklappe ausgebildeten Klappe,
- Fig. 16: eine perspektivische, schematische Ansicht einer biologischen Klappenprothese mit einem Stent und einer äußeren Solenoidspule aus einem zu einem Mäander geformten elektrisch leitenden Material,
- Fig. 17: eine perspektivische, schematische Ansicht einer biologischen Herzklappe in einem Stent mit einer äußeren Zylinderspule,
- Fig. 18: eine perspektivische, schematische Ansicht einer künstlichen Klappenprothese mit einem Stent und einer äußeren Zylinderspule sowie einer als Deckel ausgebildeten Klappe,
- Fig. 19: eine der Figur 18 entsprechende Ansicht einer künstlichen Klappenprothese mit einer als Kugel ausgebildeten Klappe,
- Fig. 20: eine der Fig. 18 entsprechende Ansicht einer künstlichen Klappenprothese mit einer als Doppelklappe ausgebildeten Klappe,
- Fig. 21: eine perspektivische, schematische Ansicht einer künstlichen Klappenprothese in einem Stent mit einer äußeren Solenoidspule aus einem zu Mäander geformten elektrisch leitenden Material und einer als Deckel ausgebildeten Klappe,
- Fig. 22: eine der Fig. 21 entsprechende Ansicht einer künstlichen Klappenprothese mit einer als Kugel ausgebildeten Klappe,
- Fig. 23: eine der Fig. 21 entsprechende Ansicht einer künstlichen Klappenprothese mit einer als Doppelklappe ausgebildeten Klappe,
- Fig. 24: eine schematische Ansicht einer in einem Katheder angeordneten Klappenprothese,
- Fig. 25: eine schematische Ansicht einer durch einen Ballon expandierbaren Klappenprothese, und
- Fig. 26: verschiedene Darstellungen von Stents bzw. Klappenprothesen mit zellengleicher oder unterschiedlicher Größe.

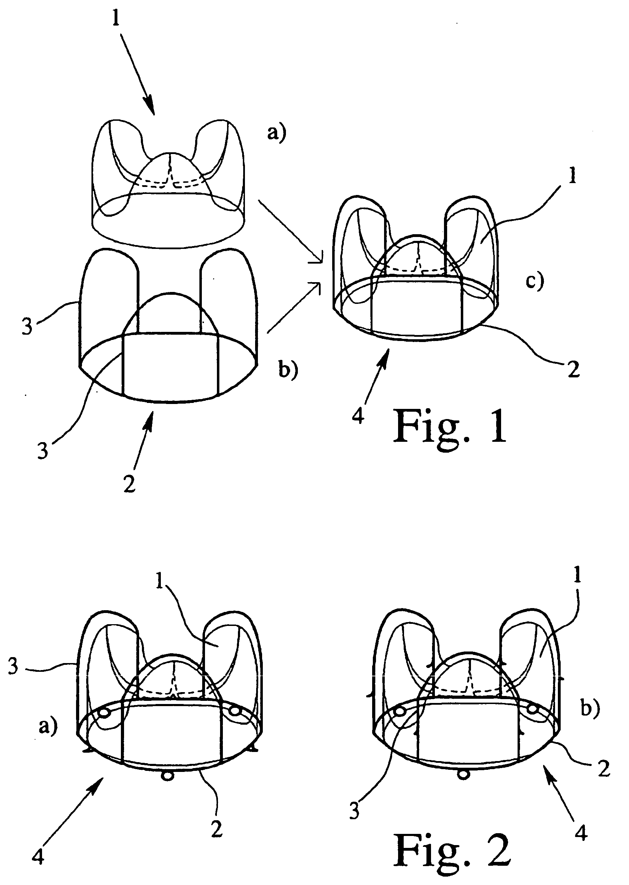

In der Darstellung a der Fig. 1 ist eine biologische Herzklappe 1 dargestellt. Die Art und Ausbildung der biologischen Herzklappe 1 an sich ist bekannt. In der Darstellung b ist eine Leiterschleife 2 gezeigt, die die Induktivität eines elektrischen Resonanzschwingkreises und damit den Resonanzschwingkreis als solchen ausbildet. Ein Vergleich der Darstellungen a und b der Fig. 1 ergibt, dass die Leiterschleife 2 als geschlossene Leiterbahn drei Schlaufen 3 ausbildet, die zusammen zylinderförmig angeordnet sind. Die Schlaufen 3 der Leiterschleife 2 sind der Form der biologischen Herzklappe 1 angepasst.

Die Darstellung c aus Fig. 1 zeigt die biologische Klappenprothese 4, wobei die Leiterschleife 2 bzw. der Schwingkreis in die biologische Herzklappe integriert sind bzw. die biologische Herzklappe 1 in die Leiterschleife 1 eingesetzt ist. Im Einzelnen nicht dargestellt ist, dass die Leiterschleife 2 wenigstens eine Kapazität aufweist.

Die in Fig. 2 dargestellten Klappenprothesen 4 unterscheiden sich von der in Fig. 1 c) dargestellten Klappenprothese 4 dadurch, dass Verankerungsmittel in Form von Ösen und Haken vorgesehen sind. Bei der Ausführungsform gemäß Fig. 2 a) befinden sich Haken und Ösen lediglich arm umlaufenden Ringbereich der Leiterschleife 2, während bei der Ausführungsform gemäß Fig. 2 b) Verankerungsmittel vorliegend in Form von Haken ebenfalls an den Schlaufen vorgesehen sind.

Nicht dargestellt ist im Übrigen, dass die Herzklappenprothese statt dreier Schlaufen auch nur zwei Schlaufen haben kann, um damit an eine Doppelsegelklappe angepasst zu sein.

Weiterhin versteht es sich, dass die in Fig. 2 dargestellten Verankerungsmittel beispielsweise in Form von Haken und/oder Ösen bei allen nachfolgend dargestellten Leiterschleifen realisiert sein können, auch wenn dies im Einzelnen nicht dargestellt ist.

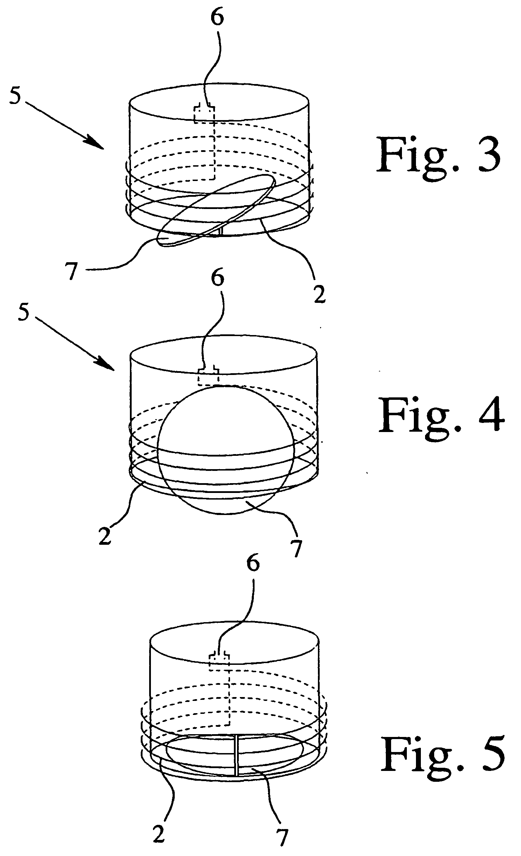

In Fig. 3 ist eine künstliche Klappenprothese 5 mit einer Leiterschleife 2 auf Basis einer Solenoidspule dargestellt. Der diesbezügliche Schwingkreis weist eine Kapazität 6 auf. Die künstliche Klappenprothese 5 weist eine als Deckel ausgebildete Klappe 7 auf. Im dargestellten Ausführungsbeispiel bildet die Leiterschleife 2 das tragende Klappengerüst der Klappenprothese 5. In diesem Zusammenhang darf darauf hingewiesen werden, dass es sich bei den einzelnen Darstellungen lediglich um schematische schaltbildartige Abbildungen der betreffenden Klappenprothesen 4, 5 handelt.

Die in Fig. 4 dargestellte künstliche Klappenprothese 5 entspricht der in Fig. 3 dargestellten Klappenprothese 5, wobei jedoch als Klappe 7 eine Kugel vorgesehen ist.

Die in Fig. 5 dargestellte künstliche Klappenprothese 5 entspricht ebenfalls der in Fig. 3 dargestellten Klappenprothese 5, wobei als Klappe 7 jedoch eine zweiflüglige Klappe nach dem Zweiklappenprinzip, die nachfolgend als Doppelklappe bezeichnet wird, vorgesehen ist.

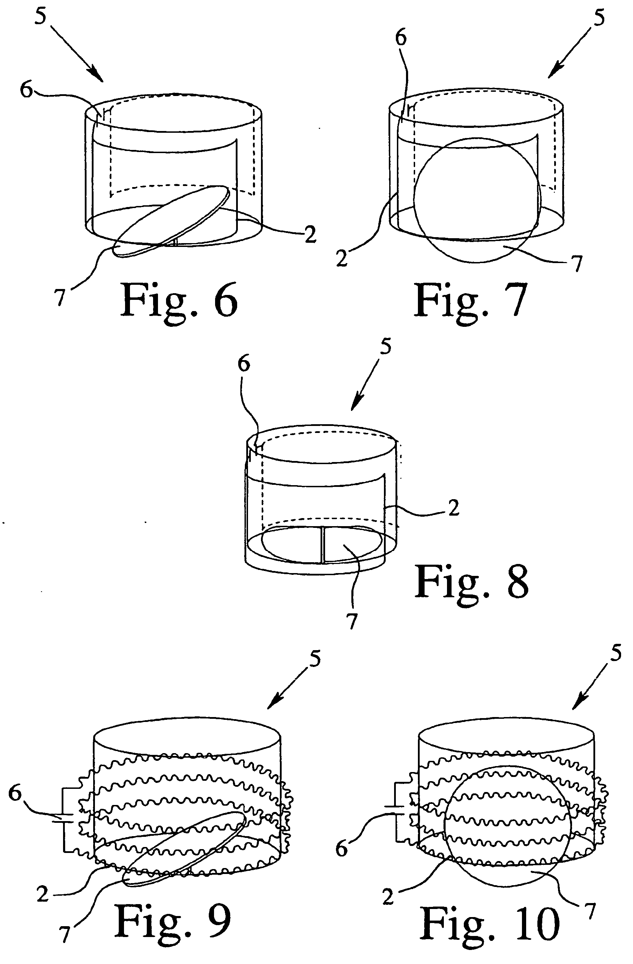

Die in Fig. 6 dargestellte Klappenprothese 5 entspricht im Wesentlichen der in Fig. 3 dargestellten Klappenprothese 5, wobei jedoch statt einer Solenoidspule eine Sattelspule vorgesehen ist.

Die in Fig. 7 dargestellte Klappenprothese 5 entspricht der in Fig. 6 dargestellten, wobei jedoch als Klappe 7 eine Kugel vorgesehen ist.

Die in Fig. 8 dargestellte Klappenprothese 5 entspricht der in Fig. 6 dargestellten, wobei jedoch als Klappe 7 eine Doppelklappe vorgesehen ist.

Bei der in Fig. 9 dargestellten künstlichen Klappenprothese 5 ist ein integrierter Resonanzschwingkreis auf Basis einer Solenoidspule dargestellt, wie dies auch aus Fig. 3 hervorgeht. Im Unterschied zu der Klappe gemäß Fig. 3 ist die Leiterschleife 2 mäanderförmig ausgebildet. Die Mäanderform des Leiterdrahtes ermöglicht es in besonders einfacher Weise, die Klappenprothese 5 zusammenzufalten.

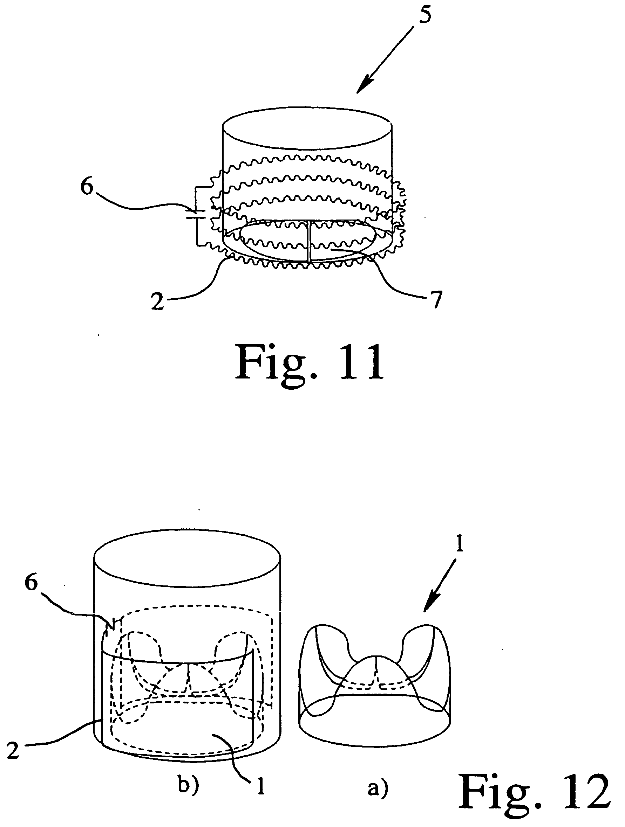

Die Klappe gemäß Fig. 10 entspricht der Klappe gemaß. Fig. 6, wobei als Klappe 7 jedoch eine Kugel vorgesehen ist. Bei der in Fig. 11 gezeigten Klappe ist im Unterschied zu der in Fig. 10 dargestellten Klappe statt der Kugel eine Doppelklappe vorgesehen.

In Fig. 12 ist in der Darstellung a eine biologische Herzklappe 1 gezeigt. Die Herzklappe 1 ist, wie sich aus der Darstellung b ergibt, in einen Stent 8 integriert. Im Übrigen ist als Träger der Herzklappe 1 und damit als Gerüst für die Herzklappe 1 eine als Sattelspule ausgebildete Leiterschleife 2 vorgesehen, die eine Kapazität 6 aufweist. Die Höhe der Leiterschleife 6 entspricht der Höhe der Herzklappe 1, so dass alle relevanten Bereiche über die Leiterschleife 2 erfasst werden können. Im Übrigen versteht es sich, dass zusätzlich zu der Sattelspule eine weitere Leiterschleife 2 vorgesehen sein kann, die in Fig. 1b dargestellt ist. Dabei können die einzelnen Spulen aus unterschiedlichen Leiterschleifen 2 gebildet sein oder aus einer einzigen Leiterschleife 2.

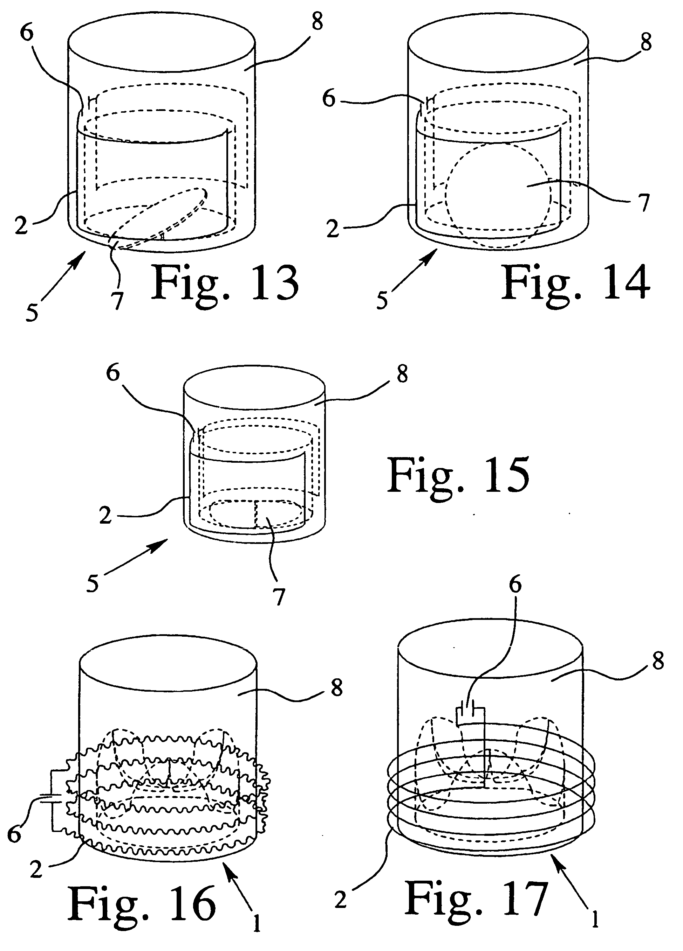

Die Ausführungsformen der Fig. 13 bis 15 entsprechen im Wesentlichen der Ausführungsform der Fig. 12, wobei es sich bei den in den Fig. 13 bis 15 dargestellten Ausführungsformen um künstliche Klappenprothesen 5 handelt, die in einem Stent 8 angeordnet sind. Die jeweilige Leiterschleife 2 ist jeweils als Sattelspule ausgebildet und innerhalb des Stents 8 angeordnet. Die Leiterschleife 2 bildet jeweils den Träger der Klappenprothese 5, wobei als Klappe 7 bei der Ausführungsform gemäß Fig. 13 ein Deckel, bei der Ausführungsform gemäß Fig. 14 eine Kugel und bei der Ausführungsform gemäß Fig. 15 eine Doppelklappe vorgesehen ist.

In den Fig. 16 bis 23 sind jeweils Ausführungsformen dargestellt, bei denen die jeweilige Klappenprothese 4, 5 einen Stent 8 aufweist, in den eine biologische Herzklappe 1 oder aber eine künstliche Herzklappe 9 als Klappenmechanismus integriert ist. Der Stent 8 weist jeweils eine Hülse aus einem biokompatiblen Material auf. Im Übrigen ist der Stent 8 in Funktionshöhe der jeweiligen Klappe von mindestens einer Leiterschleife 2, die die Induktivität eines elektrischen Resonanzschwingkreises ausbildet, umschlossen. Dabei ist es bei allen, auch den zuvor dargestellten Ausführungsformen so, dass die Höhe der jeweiligen Spule bzw. die Höhenerstreckung der Leiterschleife derart, dass sich ein solches relevantes Spulenfeld ergibt, dass die jeweilige Klappe auch während der Funktion im Wesentlichen vollständig eingeschlossen ist. Das Verhältnis des Durchmessers der Prothesenöffnung zur Höhe der jeweiligen Spule liegt zwischen 4 : 1 bis 0,5 : 1.

Auch bei den Fig. 16 bis 23 handelt es sich lediglich um schematische Darstellungen. Bei allen dargestellten Ausführungsformen sind folgende Optionen möglich:
1. Der Stent 8 weist ein nicht dargestelltes, separates Traggerüst, das den Stent 8 aufspannt und offenhält, auf. Die Leiterschleife 2 ist außen um die Hülse des Stents 8 und das Traggerüst im Bereich der Herzklappe herumgeführt.
2. Die Leiterschleife 2 bildet zumindest einen Teil des Traggerüstes oder tragende Bereiche des Gerüstes des Stents 8 aus. Hierbei übernimmt dann die Leiterschleife 2 nicht nur die Funktion des Resonanzschwingkreises, sondern auch die Gerüstfunktion des Stents.
3. Die Leiterschleife 2 ist nicht nur um den Stent 8 im relevanten Bereich gewickelt, sondern dient auch zur Befestigung der biologischen Herzklappe 1 bzw. der künstlichen Herzklappe 9. Letztlich ist die Herzklappe 1, 9 als Klappenmechanismus mit der Leiterschleife, 2 oder Teilen der Leiterschleife 2 vernäht. In diesem Falle übernimmt die Leiterschleife 2 die zusätzliche Funktion der Befestigung der Herzklappe 1, 9 innerhalb der Hülse des Stents 8. Dabei können dann nach außen geführte Bereiche der Leiterschleife 2 Haken, Ösen oder andere Verankerungsmittel aufweisen, um die Befestigung des Stents 8 an der Implantationsstelle zu erleichtern.
4. Darüber hinaus sind Kombinationen aus zwei oder drei der vorgenannten Möglichkeiten gegeben. So kann die Leiterschleife 2 zusätzlich zu der Alternative 3 auch, zumindest im relevanten Bereich der Herzklappe 1,9, die Traggerüstfunktion des Stents 8 erfüllen.

Die Fig. 16 und 17 zeigen jeweils eine biologische Herzklappe 1, die in den Stent 8 eingesetzt ist. Im relevanten Bereich, d. h. im Bereich der Herzklappe 1, befindet sich bei der Ausführungsform gemäß Fig. 16 eine Solenoidspule mit einer mäanderförmig geformten Leiterschleife 2, während bei der Ausführungsform gemäß Fig. 17 die Spule als Zylinderspule ausgeführt ist.

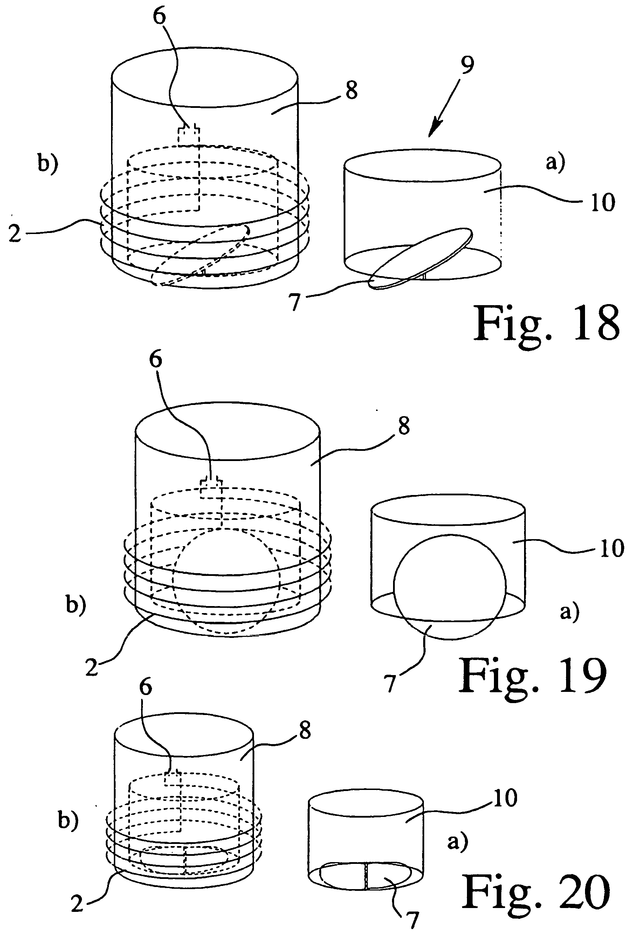

Bei der in Fig. 18 dargestellten Ausführungsform ist eine künstliche Herzklappe 9 vorgesehen, die einen Träger 10 aufweist, der zur Lagerung der Klappe 7 dient, was in Fig. 18a dargestellt ist. Bei der Darstellung gemäß Fig. 18b ist die künstliche Herzklappe 9 in den Stent 8 eingesetzt, wobei die Leiterschleife 2 eine Zylinderspule mit Kapazität 6 bildet.

Die in Fig. 19 dargestellte Ausführungsform entspricht im Wesentlichen der in Fig. 18 dargestellten Ausführungsform, wobei als Klappe 7 eine Kugel vorgesehen ist. Bei der in Fig. 20 dargestellten Ausführungsform ist hingegen als Klappe 7 eine Doppelklappe vorgesehen.

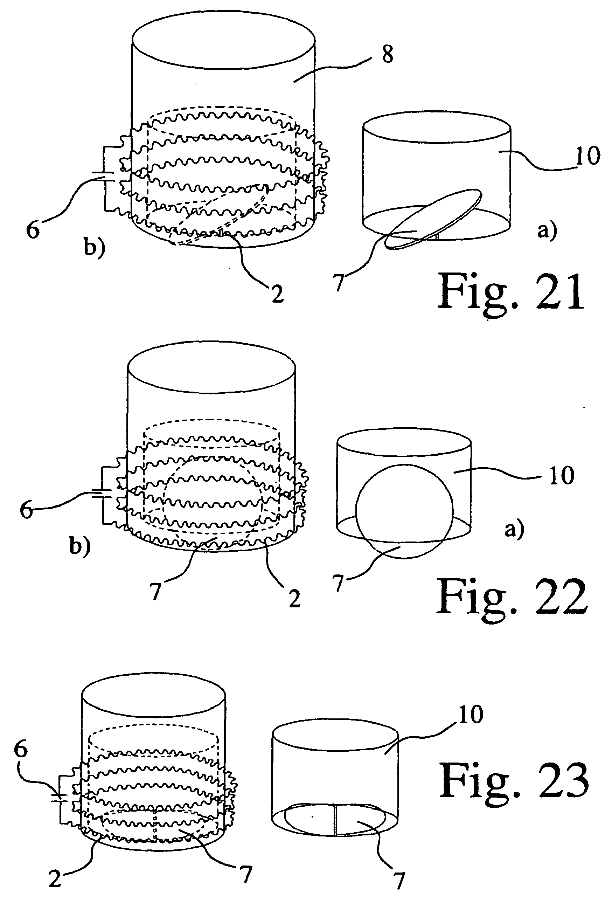

Die in Fig. 21 dargestellte Ausführungsform entspricht der in Fig. 18 dargestellten Ausführungsform, wobei die Leiterschleife 2 als Solenoidspule und die Leiterbahn 2 als solche mäanderförmig ausgebildet sind.

Während bei der Ausführungsform gemäß Fig. 21 ein Deckel als Klappe 7 vorgesehen ist, ist bei der Ausführungsform gemäß Fig. 22 eine Kugel als Klappe vorgesehen. Demgegenüber ist bei der Ausführungsform gemäß Fig. 23 als Klappe eine Doppelklappe vorgesehen. Ansonsten bestehen zwischen den Ausführungsformen gemäß den Fig. 21, 22 und 23 keine Unterschiede.

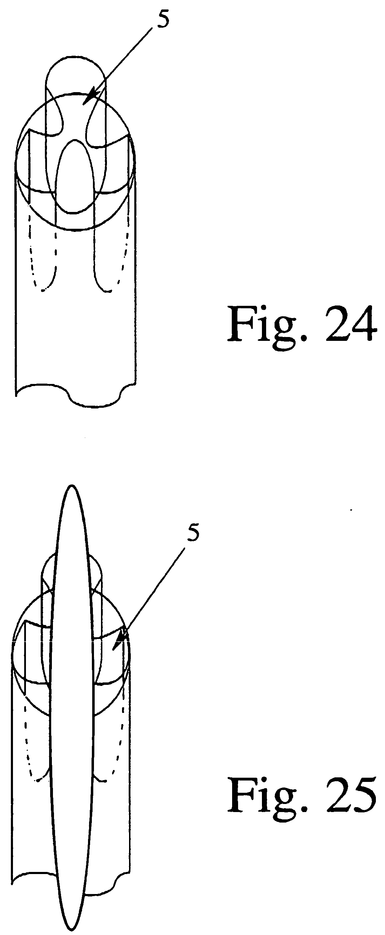

In den Fig. 24 und 25 ist schematisch das Einsetzen einer erfindungsgemäßen Klappenprothese 5 dargestellt. Bei der in Fig. 24 dargestellten Klappenprothese 5 handelt es sich um eine selbst expandierende Prothese, die nach Anordnung an der Implantationsstelle und Zurückziehen des Katheters, der die Prothese im zusammengefalteten Zustand hält, selbst expandiert. Demgegenüber wird die Prothese 5 der in Fig. 25 dargestellten Ausführungsform über einen Ballon, der innerhalb der Prothese angeordnet ist, expandiert. Hierzu wird die Prothese an die Implantationsstelle mit einem Zuführkatheter gebracht, der anschließend zurückgezogen wird. Nach Zurückziehen des Katheders faltet sich die Prothese zunächst nicht auf. Die Auffaltung wird durch Expansion des Ballons erzeugt. Hierzu wird dem Ballon ein entsprechendes Medium zugeführt. Nach der Expansion wird der Ballon aus der Prothese herausgezogen.

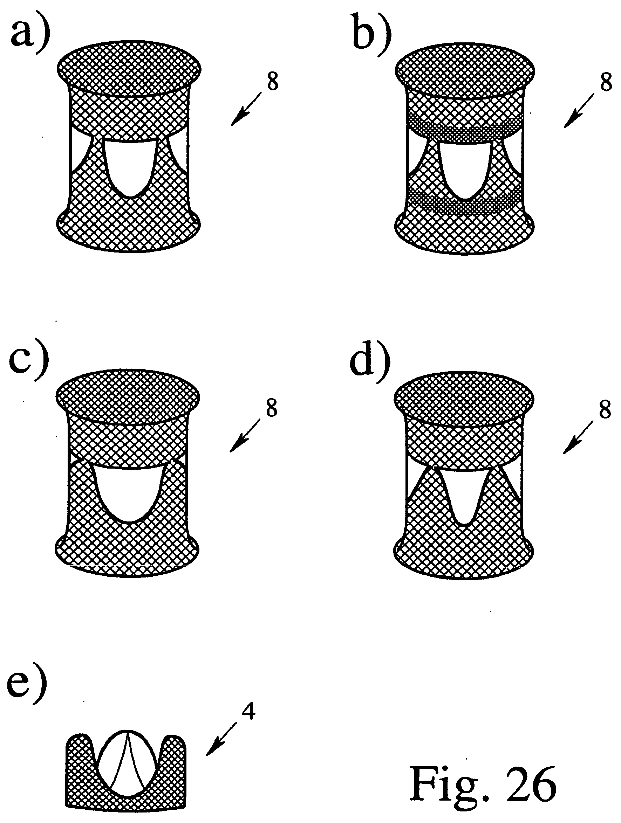

Fig. 26 zeigt verschiedene Ausführungsformen erfindungsgemäßer Klappenprothesen. Die Ausführungsformen a), b), c) und d) zeigen dabei jeweils einen Stent 8, wobei die äußere umlaufende Mantelfläche des Stents 8 gitter- oder netzförmig aufgebaut ist und dabei jeweils eine Vielzahl von Zellen mit gleicher und mit unterschiedlicher Größe aufweist. Grundsätzlich können die Zellen, die auch als Fenster oder Öffnungen bezeichnet werden können, jegliche Form haben.

Bei der Ausführungsform gemäß Fig. 26 a sind zwei Typen von Zellen vorgesehen, nämlich überwiegend kleine Zellen und im mittleren Bereich größere, etwa dreieckförmige Zellen.

Die Ausführungsform gemäß Fig. 26 b unterscheidet sich von der gemäß Fig. 26 a dadurch, dass drei Arten von Zellen mit unterschiedlichen Größen vorgesehen sind. Unterhalb und oberhalb der großen mittleren Zellen befindet sich dabei jeweils ein Streifen mit sehr kleinen Zellen.

Bei der Ausführungsform gemäß Fig. 26 c sind die mittleren Zellen relativ groß. Es sind lediglich drei große Zellen vorgesehen.

Die Ausführungsform gemäß Fig. 26 d entspricht im Wesentlichen der gemäß Fig. 26 c, wobei jedoch mehr als drei große mittlere Zellen vorgesehen sind.

Fig. 26 e zeigt eine Klappenprothese (4), die im Wesentlichen der Klappenprothese gemäß Fig. 1 c entspricht, wobei jedoch außenseitig eine Vielzahl von kleinen Zellen vorgesehen sind.

## Patentansprüche

1. Biologische oder künstliche Klappenprothese (4, 5) zur Verwendung im menschlichen oder tierischen Körper zum Ersatz einer Organklappe oder Gefäßklappe, insbesondere Herz- oder Venenklappenprothese, mit einem tragenden Klappengerüst, mit wenigstens einer Klappe (7) und mit wenigstens einer Leiterschleife (2), die die Induktivität eines elektrischen Resonanzschwingkreises ausbildet, wobei
die wenigstens eine Leiterschleife (2) das Klappengerüst und/oder die Klappe (7) und/oder tragende Bereiche des Klappengerüstes und/oder tragende Bereiche der Klappe (7) ausbildet, **dadurch gekennzeichnet, dass** das Klappengerüst und die Klappe (7) in einem selbst entfaltbaren oder mechanisch expandierbaren Stent (8) durch die Leiterschleife (2) derart fixiert sind, dass bei der Komprimierung und anschließenden Entfaltung sowohl das Klappengerüst und die Klappe (7) als auch die Leiterschleife (2) in die gewünschte Form, Position und Geometrie zurückkehren, und dass die Leiterschleife (2) gleichzeitig zur Befestigung der Klappe (7) am Stent (8) dient.

2. Klappenprothese nach Anspruch 1, **dadurch gekennzeichnet, dass** zumindest Bereiche der Leiterschleife (2) als Verankerungsmittel für eine Verankerung im umgebenden Gewebe aus dem Stent (8) herausgeführt sind.

3. Klappenprothese nach einem der vorhergehenden Ansprüche, **dadurch gekennzeichnet, dass** der Resonanzschwingkreis derart ausgebildet ist, dass der Schwingkreis eine Resonanzfrequenz im Bereich zwischen 8 bis 400 MHz besitzt, die der Frequenz eines äußeren Magnetfeldes eines MR-Tomographen entspricht.

4. Klappenprothese nach einem der vorhergehenden Ansprüche, **dadurch gekennzeichnet, dass** die Leiterschleife (2) aus einem Draht oder einem Rohr oder aus Blech geschnitten und aus einem Material mit einer geringen magnetischen Suszeptibilität und/oder einer guten elektrischen Leitfähigkeit hergestellt ist.

5. Klappenprothese nach einem der vorhergehenden Ansprüche, **dadurch gekennzeichnet, dass** die Leiterschleife (2) einen nicht leitenden Leiterträger aufweist, der mittels eines leitfähigen Materials beschichtet ist oder dass die Leiterschleife (2) einen elektrisch leitfähigen Leiterträger aufweist, der zur Verbesserung seiner elektrischen Leitfähigkeit mit einem entsprechenden Material beschichtet ist.

6. Klappenprothese nach einem der vorangehenden Ansprüche, **dadurch gekennzeichnet, dass** die Leiterschleife (2) durch eine Beschichtung der Klappenprothese mit einem leitenden Material realisiert ist.

7. Klappenprothese nach einem der vorangehenden Ansprüche, **dadurch gekennzeichnet, dass** die Leiterschleife (2) zur Einstellung einer externen parasitären Kapazität mit einem Nicht-Leiter als Isolationsschicht beschichtet ist, deren Dicke mindestens 1 Nanometer beträgt, und dass eine interne Kapazität durch die Isolationsschicht und den Abstand zwischen den Schlaufen der wenigstens einen Leiterbahn ausgebildet wird.

8. Klappenprothese nach Anspruch 7, **dadurch gekennzeichnet, dass** die Isolationsschicht eine Dicke zwischen 1 nm bis 200 µm aufweist.

9. Klappenprothese nach einem der vorangehenden Ansprüche 7 oder 8, **dadurch gekennzeichnet, dass** die Isolationsschicht die zusätzliche Aufgabe hat, eine weitere zum Resonanzschwingkreis gehörende Spule und/oder wenigstens eine Kapazität auf oder in dem Klappengerüst zu befestigen bzw. anzubringen.

10. Klappenprothese nach einem der vorhergehenden Ansprüche, **dadurch gekennzeichnet, dass** die Leiterschleife (2) die Form einer natürlichen Herzklappe hat und wenigstens zwei Schlaufen der Leiterschleife (2) vorgesehen sind, die mit ihrem Flächen eine Zylinderform bilden, die natürliche Klappenform umschließen und in den Bereichen zwischen den Schlaufen die Abgänge des Klappengerüstes zu den Herzkranzgefäßen freihalten.

11. Klappenprothese nach einem der vorhergehenden Ansprüche, **dadurch gekennzeichnet, dass** die Leiterschleife (2) die Form einer biologischen Herzklappe hat und das mindestens zwei Schlaufen der Leiterschleife (2) vorgesehen sind, die mit ihren Flächen eine Zylinderform bilden, die die natürliche Klappenform schlaufenförmig umschließen und in den Bereichen zwischen den Schlaufen die Abgänge des Klappengerüstes zu den Herzkranzgefaßen freihalten.

12. Perkutan implantierbare und expandierbare, biologische oder künstliche Klappenprothese (4. 5) zur Verwendung im menschlichen und/oder tierischen Körper zum Ersatz einer Organklappe oder Gefäßklappe, mit mindestens einem mit einer Klappe (7) versehenen Klappenmechanismus, characterisiert durch mit einem eine Hülse aus biokompatiblen Material aufweisenden Stent (8), wobei der Stent (8) in der Funktionshöhe der Klappe von mindestens einer Leiterschleife (2), die die Induktivität eines elektrischen Resonanzschwingkreises ausbildet, umschlossen ist, wobei der Klappenmechanismus in dem Stent (8) mittels der Leiterschleife (2) an der Hülse des Stents (8) befestigt ist.

13. Klappenprothese nach Anspruch 12, **dadurch gekennzeichnet, dass** aus der Hülse ragende Bereiche der Leiterschleife (2) als Verankerungsmittel, insbesondere als Haken und/oder Ösen, ausgebildet sind.

## Claims

1. Biological or artificial valve prosthesis (4, 5) for use in the human or animal body for replacing an organ valve or vessel valve, in particular a heart valve prosthesis or venous valve prosthesis, comprising a supporting valve scaffold, comprising at least one valve (7) and comprising at least one conductor loop (2), which forms the inductor of an electrical resonant circuit, wherein
the at least one conductor loop (2) forms the valve scaffold and/or the valve (7) and/or supporting regions of the valve scaffold and/or supporting regions of the valve (7), **characterized in that** the valve scaffold and the valve (7) are fixed by way of the conductor loop (2) in a self-deployable or mechanically expandable stent (8) in such a way that, in the case of compression and subsequent deployment, both the valve scaffold and the valve (7) and also the conductor loop (2) return to the desired form, position and geometry and **in that** the conductor loop (2) simultaneously serves to attach the valve (7) at the stent (8).

2. Valve prosthesis according to Claim 1, **characterized in that** at least portions of the conductor loop (2) are guided out of the stent (8) as anchoring means for fixation in the surrounding tissue.

3. Valve prosthesis according to one of the preceding claims, **characterized in that** the resonant circuit is embodied in such a way that the resonant circuit has a resonant frequency in the range between 8 and 400 MHz, which corresponds to the frequency of an outer magnetic field of an MRI scanner.

4. Valve prosthesis according to one of the preceding claims, **characterized in that** the conductor loop (2) is cut out of a wire or a pipe or foil and produced from a material with a low magnetic susceptibility and/or good electrical conductivity.

5. Valve prosthesis according to one of the preceding claims, **characterized in that** the conductor loop (2) has a nonconductive conductor carrier, which is coated by a conductive material, or **in that** the conductor loop (2) has an electrically conductive conductor carrier, which is coated by an appropriate material for improving the electrical conductivity thereof.

6. Valve prosthesis according to one of the preceding claims, **characterized in that** the conductor loop (2) is realized by a coating of the valve prosthesis with a conductive material.

7. Valve prosthesis according to one of the preceding claims, **characterized in that** the conductor loop (2) is coated with a non-conductor as an insulation layer for setting an external parasitic capacitance, the thickness of which insulation layer is at least 1 nanometre, and **in that** an internal capacitor is formed by the insulation layer and the distance between the loops of the at least one conductor track.

8. Valve prosthesis according to Claim 7, **characterized in that** the insulation layer has a thickness of between 1 nm and 200 µm.

9. Valve prosthesis according to either one of the preceding Claims 7 and 8, **characterized in that** the insulation layer additionally has the object of fastening or attaching a further coil, which is part of the resonant circuit, and/or at least one capacitor on or in the valve scaffold.

10. Valve prosthesis according to one of the preceding claims, **characterized in that** the conductor loop (2) has the form of a natural heart valve and at least two loops of the conductor loop (2) are provided, which form a cylindrical form with the surfaces thereof, surround the natural valve form and keep the outflows of the valve scaffold to the coronary arteries free in the regions between the loops.

11. Valve prosthesis according to one of the preceding claims, **characterized in that** the conductor loop (2) has the form of a biological heart valve and **in that** at least two loops of the conductor loop (2) are provided, which form a cylindrical form with the surfaces thereof, which surround the natural valve form in a loop-shaped manner and keep the outflows of the valve scaffold to the coronary arteries free in the regions between the loops.

12. Percutaneously implantable and expendable, biological or artificial valve prosthesis (4, 5) for use in the human and/or animal body for replacing an organ valve or vessel valve, comprising at least one valve mechanism provided with a valve (7) **characterized by** a stent (8) having a sleeve made out of biocompatible material, wherein the stent (8) is surrounded at the functional height of the valve by at least one conductor loop (2), which forms the inductor of an electrical resonant circuit, wherein the valve mechanism is fastened to the sleeve of the stent (8) in the stent (8) by means of the conductor loop (2).

13. Valve prosthesis according to Claim 12, **characterized in that** the region of the conductor loop (2) projecting out of the sleeve is embodied as anchoring means, in particular as hooks and/or ears.

## Revendications

1. Prothèse de valve artificielle ou biologique (4, 5) destinée à une utilisation dans un corps humain ou animal en remplacement de la valve d'un organe ou d'un vaisseau, en particulier prothèse de valve cardiaque ou veineuse, comprenant une cage de valve portante, avec au moins une valve (7) et au moins une boucle conductrice (2) qui constitue l'inductance d'un circuit résonant électrique,
l'au moins une boucle conductrice (2) constituant la cage de valve et/ou la valve (7) et/ou des régions portantes de la cage de valve et/ou des régions portantes de la valve (7),
**caractérisée en ce que** la cage de valve et la valve (7) sont fixées par la boucle conductrice (2) dans une endoprothèse (8) auto-dépliable ou déployable mécaniquement de telle sorte que lors de la compression et du dépliement subséquent, la cage de valve et la valve (7) ainsi que la boucle conductrice (2) reviennent à la forme, la position et la géométrie souhaitées, et que la boucle conductrice (2) serve simultanément à la fixation de la valve (7) à l'endoprothèse (8).

2. Prothèse de valve selon la revendication 1, **caractérisée en ce qu'**au moins certaines régions de la boucle conductrice (2) sont guidées hors de l'endoprothèse (8) en tant que moyen d'ancrage pour un ancrage dans le tissu environnant.

3. Prothèse de valve selon l'une quelconque des revendications précédentes, **caractérisée en ce que** le circuit résonant électrique est réalisé de telle sorte que le circuit résonant possède une fréquente de résonance dans la plage de 8 à 400 MHz, qui correspond à la fréquente d'un champ magnétique extérieur d'un tomographe RM.

4. Prothèse de valve selon l'une quelconque des revendications précédentes, **caractérisée en ce que** la boucle conductrice (2) est découpée dans un fil métallique ou un tube ou est découpée dans une tôle et est fabriquée à partir d'un matériau ayant une plus faible susceptibilité magnétique et/ou une bonne conductibilité électrique.

5. Prothèse de valve selon l'une quelconque des revendications précédentes, **caractérisée en ce que** la boucle conductrice (2) présente un support de conducteur non conducteur, qui est revêtu d'un matériau conducteur ou **en ce que** la boucle conductrice (2) présente un support de conducteur électriquement conducteur, qui est revêtu d'uh matériau correspondant en vue d'améliorer sa conductibilité électrique.

6. Prothèse de valve selon l'une quelconque des revendications précédentes, **caractérisée en ce que** la boucle conductrice (2) est réalisée par un revêtement de la prothèse de valve avec un matériau conducteur.

7. Prothèse de valve selon l'une quelconque des revendications précédentes, **caractérisée en ce que** la boucle conductrice (2) est revêtue d'un non-conducteur en tant que couche isolante pour ajuster une capacité parasite externe, la couche isolante ayant une épaisseur d'au moins 1 manomètre, et **en ce qu'**une capacité interne est réalisée par la couche isolante et la distance entre les boucles de l'au moins une piste conductrice.

8. Prothèse de valve selon la revendication 7, **caractérisée en ce que** la couche isolante présente une épaisseur comprisse entre 1 nm et 200 µm.

9. Prothèse de valve selon l'une quelconque des revendications 7 ou 8, **caractérisée en ce que** la couche isolante a pour fonction supplémentaire de fixer ou de monter une bobine supplémentaire appartenant au circuit résonant et/ou au moins un capaciteur sur ou dans la cage de valve.

10. Prothèse de valve selon l'une quiconque des revendications précédentes, **caractérisée en ce que** la boucle conductrice (2) a la forme d'une valve cardiaque naturelle et au moins deux boucles de la boucle conductrice (2) sont prévues, lesquelles forment avec leurs surfaces une forme cylindrique, enveloppent la forme de valve naturelle et, dans les régions entres les boucles, maintiennent libres les sorties de la cage de valve vers les vaisseaux du coeur.

11. Prothèse de valve selon l'une quelconque des revendications précédentes, **caractérisée en ce que** la boucle conductrice (2) a la forme d'une valve cardiaque biologique et au moins deux boucles de la boucle conductrice (2) sont prévues, lesquelles forment avec leurs surfaces une forme cylindrique, enveloppent la forme de valve naturelle en forme de boucle et, dans les régions entre les boucles, maintiennent libres les sorties de la cage de valve vers les vaisseaux du coeur.

12. Prothèse de valve artificielle ou biologique (4, 5), implantable et déployable par voie sous-cutanée, destinée à une utilisation dans un corps humain et/ou animal en remplacement de la valve d'un organe ou d'un vaisseau, comprenant au moins un mécanisme de valve pourvu d'une valve (7), **caractérisée par** une endoprothèse (8) présentant une gaine en matériau bio-compatible, l'endoprothèse (8), à la hauteur de la valve, étant entourée par au moins une boucle conductrice (2) qui constitue l'inductance d'un circuit résonant électrique, le mécanisme de valve dans l'endoprothèse (8) étant fixé au moyen de la boucle conductrice (2) à la gaine de l'endoprothèse (8).

13. Prothèse de valve selon la revendication 12, **caractérisée en ce que** des régions de la boucle conductrice (2) saillant hors de la gaine sont réalisées sous forme de moyens d'ancrage, en particulier sous forme de crochets et/ou d'oeillets.
